# EUROPEAN PATENT SPECIFICATION

(11) **EP 1 645 236 B1**
(45) Date of publication and mention of the grant of the patent: **11.08.2010**
(21) Application number: 05021944.3
(22) Date of filing: 07.10.2005
(51) Int. Cl.: A61B 18/12

(54) **Devices for detecting heating under a patient return electrode**
Geräte zur Messung der Erwärmung unter einer Neutralelektrode
Outils pour la détection du chauffage au- dessous d'une électrode neutre

(30) Priority: 08.10.2004 US 616970 P; 03.10.2005 US 242475
(43) Date of publication of application: 12.04.2006
(60) Divisional application: 10166488.6
(73) Proprietor: Covidien AG, 8212 Neuhausen am Rheinfall (CH)
(72) Inventor: Eggleston, Jeffrey L., Broomfield, Colorado 80020 (US)
(74) Representative: HOFFMANN EITLE

(56) References cited:
- DE-A1- 19 717 411
- US-A- 4 343 308
- US-A- 4 741 334
- US-B1- 6 258 085

## Description

### BACKGROUND

### 1. Technical Field

The present disclosure relates to safety devices and methods during electrosurgery, and more particularly the present disclosure relates to devices for detecting, determining and/or approximating the probability of patient burn under a return electrode in a monopolar electrosurgical system.

### 2. Background of Related Art

During electrosurgery, a source or active electrode delivers a first electrical potential, such as radio frequency energy, to an operative site and a return electrode carries a second electrical potential back to the electrosurgical generator. In monopolar electrosurgery, the source electrode is typically the hand-held instrument placed by the surgeon at the surgical site. By varying the energy and current density, this electrode will create a desired surgical effect, e.g., cutting, coagulating or ablating tissue. A patient return electrode is typically placed at a remote location from the source electrode and is typically in the form of a pad adhesively adhered to the patient's skin.

The return electrode has a large patient contact surface area to minimize heating at the contact surface. Smaller surface areas tend to have greater current densities and greater heat intensity. A larger surface contact area is desirable to reduce heat intensity. Return electrodes are sized based on assumptions of the maximum anticipated current during a particular surgery and the duty cycle (the percentage of time the generator is on) during the procedure. The first types of return electrodes were in the form of large metal plates covered with conductive jelly. Later, adhesive electrodes were developed with a single metal foil covered with conductive jelly or conductive adhesive. However, one problem with these adhesive electrodes was that if a portion peeled from the patient, the contact area of the electrode with the patient decreased, thereby increasing the current density at the adhered portion and, in turn, increasing the heat to the tissue.

To address this problem, split return electrodes and hardware circuits, generically called "Return Electrode Contact Quality Monitors" (RECQMs), were developed. These split electrodes consist of two separate conductive foils. The hardware circuit uses an AC signal between the two electrode halves to measure the impedance therebetween. The impedance measurement is indicative of how well the return electrode is adhered to the patient, i.e., the impedance between the two halves is directly related to the area of patient contact. If during surgery, an electrode begins to peel from the patient, the impedance would increase due to the decrease in the contact area of the electrode. Current RECQMs are designed to sense this change in impedance so that when a percentage increase in impedance exceeds a predetermined value or the measured impedance exceeds a threshold level, the electrosurgical generator is shut down to reduce the chances of overheating at the return electrode site.

Although monitoring circuits in present use are effective, they do not take into account the amount of time the current is being delivered. As new surgical procedures continue to be developed that utilize higher current and higher duty cycles, increased heating of tissue under the return electrode will occur. It would therefore be advantageous to design a monitoring circuit which would also factor in the amount of time the current is being delivered in determining a probability that a patient may ultimately bum. Based on this probability determination, an alarm signal can be generated or energy supplied from the generator can be discontinued.

For example, German patent application DE 197 17 411 A1 discloses a method of monitoring the thermal load on a tissue in a patient.

U.S. Patent 6,258,085, discloses a method and system to precisely measure the current flowing in a monopolar electrosurgical circuit and calculate the heat deposited under a patient return electrode. The calculated value of heat deposited under the patient return electrode along with a calculated cooling factor enables monitoring and/or tracking of potential occurrences of burning that may take place under the return electrode.

Unfortunately, older systems and/or generators already in the field may not be equipped and/or capable of performing such monitoring.

Accordingly, the need exists for a device which can detect and/or predict heating under a patient return electrode. More particularly, the need exists for a device which can be operatively coupled to existing and/or older generators to thereby provide the existing and/or older generators with the ability to monitor potential occurrences of burning that may take place under the return electrode.

### SUMMARY

The invention is defined in claim 1 below. The dependent claims are directed to optional features and preferred embodiments.

The present disclosure relates to safety devices and methods for use during electrosurgery, and more particularly the present disclosure relates to devices for detecting, determining and/or approximating the probability of patient burn under a return electrode in a monopolar electrosurgical system.

More specifically, one embodiment of the present disclosure includes an apparatus for predicting the temperature at a return electrode in a monopolar electrosurgical system. The apparatus includes a detecting apparatus adapted to connect to a power source which, in turn, includes an analog circuit configured to sense a change of temperature in an element . A comparator is included which is configured to compare the change in the above element to a threshold value. The detecting apparatus is configured to predict skin temperature of the patient at the return electrode of the monopolar electrosurgical system without contacting the patient.

In one embodiment, the apparatus is operatively coupled between the return electrode and the electrosurgical generator. The power source typically includes a portable power supply and is adapted to operatively couple to an existing monopolar electrosurgical system.

The detecting apparatus may be configured to include a mechanism designed to produce an advisory signal or warning signal to the user if a certain condition is met or determined or prior to meeting a threshold value. For example, the advisory signal is generated prior to conditions which may cause patient burn when the temperature under the return pad is predicted to exceed about a 4 degree temperature rise.

The detecting apparatus includes a heat generating resistor and a temperature sensor. The heat generating resistor and the temperature sensor are in thermal communication and the heating and cooling properties of the apparatus are proportional to the heating and cooling properties of patient skin at the return electrode. Patient skin temperature is predicted from the temperature of the apparatus.

The present disclosure also relates to a system for predicting the temperature under a return electrode in a monopolar electrosurgical system and includes a detecting apparatus adapted to connect to a power source, a comparator configured to compare the change in temperature with a threshold value and an alarm circuit configured to produce an advisory signal. The detecting apparatus also includes an analog circuit having a heat generating resistor and a temperature sensor. The heat generating resistor and the temperature sensor are typically configured to reside in thermal communication with one another. The heating and cooling properties of the analog circuit are proportional to the heating and cooling properties of skin at the return electrode and the detecting apparatus is configured to predict skin temperature of the patient at the return electrode of the monopolar electrosurgical system without contacting the patient. The alarming circuit alerts the user when the predicted skin temperature exceeds the threshold value.

In yet another embodiment, the power source includes a portable power supply. An advisory signal may also be generated when the temperature under the return pad is predicted to exceed about a 4 degree temperature rise. The system may also be configured to adaptively or operatively couple to an existing monopolar electrosurgical system.

### BACKGROUND

### 1. Technical Field

The present disclosure relates to safety devices and methods during electrosurgery, and more particularly the present disclosure relates to devices for detecting, determining and/or approximating the probability of patient burn under a return electrode in a monopolar electrosurgical system.

### BRIEF DESCRIPTION OF THE DRAWINGS

The accompanying drawings, which are incorporated in and constitute a part of the specification, illustrate embodiments of the present disclosure and, together with the detailed description of the embodiments given below, serve to explain the principles of the disclosure.

FIG. 1 is a schematic illustration of a monopolar electrosurgical system;

FIG. 2 is a schematic block diagram of an electrosurgical system including a device for detecting heating under a patient return electrode, in accordance with the present disclosure; and

FIG. 3 is a schematic block diagram further illustrating the device for detecting heating under a patient return electrode in accordance with the present disclosure.

### DETAILED DESCRIPTION

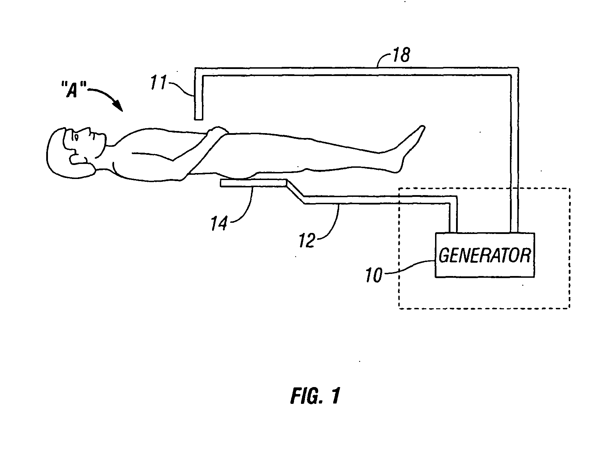

FIG. 1 is a schematic illustration of a monopolar electrosurgical system. The surgical instrument for treating tissue at the surgical site is designated by reference numeral 11. Electrosurgical energy is supplied to instrument 11 by generator 10 via cable 18 to produce an electrosurgical effect (e.g., cut, coagulate, etc.) on tissue of patient "A". A return electrode, designated by reference numeral 14, is shown placed under patient "A" to return the energy from patient "A" back to generator 10 via cable 12. Return electrode 14 is typically in the form of a pad which is adhesively attached to the skin of patient "A".

The area of return electrode 14 that adheres to patient "A" is important since area affects the current density of the signal that heats the tissue. The smaller the contact area between return electrode 14 and the tissue of patient "A", the greater the current density and heat at return electrode 14 and patient tissue site. Conversely, the greater the contact area between return electrode 14 and the tissue of patient "A", the smaller the current density and the heat at the return electrode and patient tissue site.

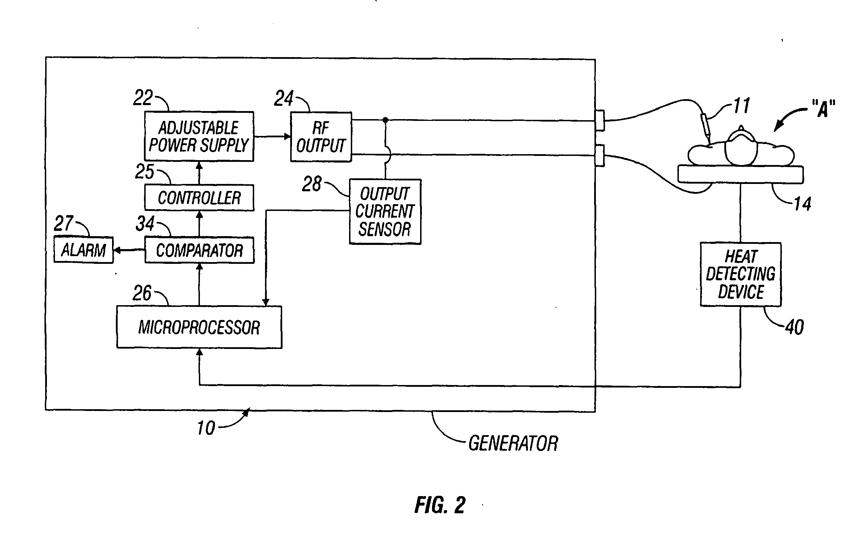

With reference to FIG. 2, electrosurgical generator 10 includes a microprocessor 26, an adjustable power supply 22, such as a high voltage supply, for producing RF current, and an RF output stage 24 electrically connected to the power supply 22 for generating an output voltage and output current for transmission to instrument 11. Power supply 22 is adjusted by controller 25 dependent on the calculated probability of patient burn.

Microprocessor 26 includes a plurality of input ports. One input port is in electrical communication with an output current sensor 28 which measures the output current of RF output stage 24 being transmitted to patient "A". During the surgical procedure, generator 10 is activated at set or varying time intervals, with intermittent shut down time intervals to allow the tissue to naturally cool as the blood flow of patient "A" dissipates heat.

Another input port of microprocessor 26 is in electrical communication with a device 40 for detecting heating under return electrode 14. Device 40 detects heating between return electrode 14 and the skin of patient "A". Device 40 is desirably operatively couplable between return electrode 14 and microprocessor 26 of generator 10.

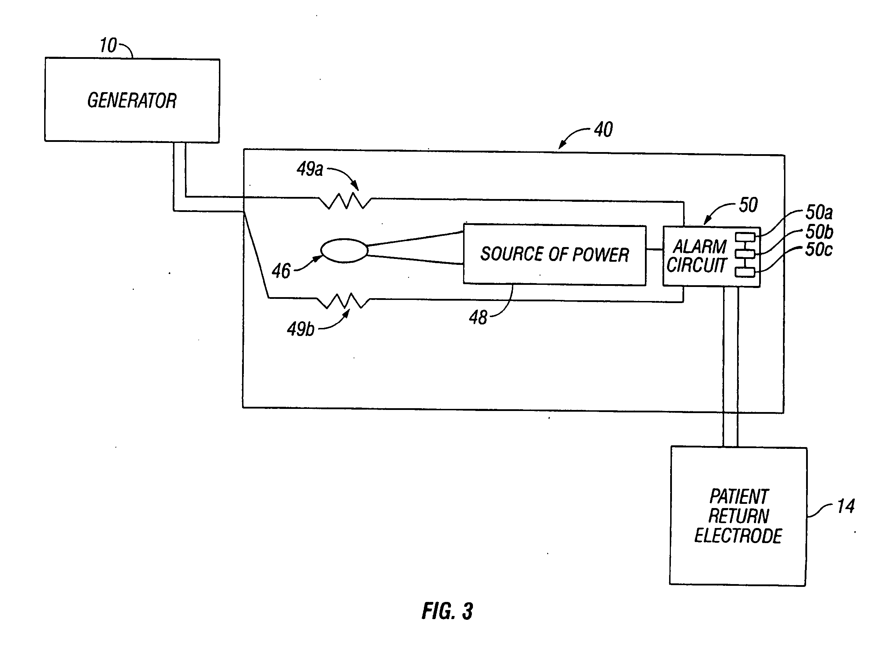

As best seen in FIG. 3, heat detecting device 40 includes at least a pair of resistors 44a, 44b, connected in series between generator 10 and return electrode 14. Resistors 44a, 44b are sized to generate heat at a rate substantially equal to or substantially proportional to the rate of heat generation which would occur at the interface between the skin of patient "A" and a return pad 14 properly applied to the skin of patient "A".

Heat detecting device 40 further includes at least one heat sink, temperature sensor and/or any other temperature measuring device 46 configured to measure temperature. Temperature measuring device 46 is located in close proximity to resistors 44a, 44b such that temperature measuring device 46 can sense and/or measure a change in temperature of resistors 44a, 44b.

By properly sizing and/or selecting appropriate resistors 44a, 44b and temperature measuring devices 46, the cooling effect and/or the heating effect experienced by the skin of patient "A" can be approximated. In other words, as the skin of patient "A" undergoes an increase (heating effect) or a decrease (cooling effect) in temperature, temperature measuring device 46 undergoes a corresponding and/or a proportional amount of temperature decrease/increase. Although each individuals threshold for temperature is different, the present device would be designed to predict when the temperature under the return electrode approaches but does not exceed the 6 degrees of temperature rise normally allowed by the applicable safety standard for the average patient. It may be desirable to select 4 degrees rise as the alarm point for instance.

Temperature measuring device 46 is operatively connected to a source of power 48, typically a portable power supply such as a battery-type source of power 48. By providing a battery-type source of power 48, heat detecting device 40 can be universally used around the world, irrespective of the configuration of the electrical outlets and the like. Moreover, a battery-type source of power 48 enables heat detecting device 40 to be independent and/or stand alone (i.e., heat detecting device 40 does not have to be plugged into and/or otherwise connected to a source of power).

Heat detecting device 40 can further include an alarm circuit 50 operatively connected to (e.g., in electrical communication with) temperature sensing device 46 for generating an advising signal when a threshold value is reached and/or surpassed. Alarm circuit 50 includes low power analog circuitry 50a to extend battery life and make device 40 cost effective. Alarm circuit 50 includes a comparator 50b in electrical communication with analog circuitry 50a. In operation, the voltage produced by analog circuitry 50a is compared to threshold values supplied to or present in comparator 50b.

Accordingly, if the voltage produced by analog circuitry 50a exceeds the threshold value present in comparator 50b, a signal is sent to an alarm 50c to generate an advisory signal (e.g., an audible, a visual and/or a tactile signal) as a warning to the user. It is envisioned that alarm circuit 50 can function as a two step set point, wherein a first alarm is activated when the temperature is relatively high but not yet high enough to result in patient burn, and a second alarm is activated when the temperature exceeds the threshold values present in comparator 50b to thereby indicate to the user that patient burn is possible.

Accordingly, since heat detecting device 40 can be operatively connected to existing generators and/or operatively connected to generators which do not include systems for detecting heat generation against the skin of a patient; continued use of such generators is possible.

While the above description contains many specifics, those specifics should not be construed as limitations on the scope of the disclosure, but merely as exemplifications of preferred embodiments thereof. Those skilled in the art will envision many other possible variations that are within the scope of the disclosure as defined by the claims appended hereto.

## Claims

1. A system for predicting the temperature under a return electrode (14) in a monopolar electrosurgical system, comprising:
a detecting apparatus (40) adapted to connect to a power source (48) including:
an analog circuit (50a) including:
a heat generating resistor (44a, 44b) connected in series between a generator (10) and said return electrode (14) and
a temperature sensor (46), configured to sense a change in temperature of said resistor (44a, 44b);
wherein said heat generating resistor (44a, 44b) and said temperature sensor (46) are in thermal communication;
a comparator (50b) configured to compare said change in temperature with a threshold value; and
an alarm circuit (50) configured to produce an advisory signal;
wherein the heating and cooling properties of said analog circuit (50a) are proportional to the heating and cooling properties of skin at the return electrode (14), wherein said detecting apparatus (40) is configured to predict skin temperature of the patient at said return electrode (14) of said monopolar electrosurgical system without contacting the patient, and wherein said alarm circuit (50) is configured alert the user when the predicted skin temperature exceeds the threshold value.

2. The apparatus of claim 1, wherein said power source (48) comprises a portable power supply.

3. The system of claim 1, wherein said advisory signal is generated when the temperature under the return pad (14) is predicted to exceed about a 4 degrees temperature rise.

4. The system of claim 1, wherein the system is adapted to operatively couple to an existing monopolar electrosurgical system.

5. The system of claim 1, wherein the apparatus is operatively coupled between the return electrode (14) and the generator (10).

6. The system of claim 1, wherein said advisory signal is generated prior to conditions which may cause patient burn.

## Patentansprüche

1. System zum Prognostizieren einer Temperatur unter einer Rückführelektrode (14) in einem monopolaren elektrochirurgischen System, umfassend:
eine Detektionsvorrichtung (40), die angepasst ist, um mit einer Leistungsquelle (48) verbunden zu sein,
enthaltend:
eine analoge Schaltung (50a), enthaltend:
einen Wärme erzeugenden Widerstand (44a, 44b), der in Reihe zwischen einem Generator (10) und der Rückführelektrode (14) angeschlossen ist, und
einen Temperatursensor (46), der ausgestaltet ist, um eine Temperaturänderung des Widerstands (44a, 44b) zu messen;
wobei der Wärme erzeugende Widerstand (44a, 44b) und der Temperatursensor (46) in thermischer Verbindung stehen;
einen Komparator (50b), der ausgestaltet ist, um die Temperaturänderung mit einem Schwellenwert zu vergleichen; und
eine Alarmschaltung (50), die ausgestaltet ist, um ein Warnsignal zu erzeugen;
wobei die Erwärmungs- und Kühleigenschaften der analogen Schaltung (50a) proportional zu den Erwärmungs- und Kühleigenschaften von Haut an der Rückführelektrode (14) sind, wobei die Detektionsvorrichtung (40) ausgestaltet ist, um die Hauttemperatur des Patienten an der Rückführelektrode (14) des monopolaren elektrochirurgischen Systems zu prognostizieren, ohne den Patienten zu kontaktieren, und wobei die Alarmschaltung (50) ausgestaltet ist, um den Benutzer zu alarmieren, wenn die prognostizierte Hauttemperatur den Schwellenwert überschreitet.

2. Vorrichtung nach Anspruch 1, wobei die Leistungsquelle (48) eine tragbare Leistungsquelle umfasst.

3. System nach Anspruch 1, wobei das Warnsignal erzeugt wird, wenn die Temperatur unter dem Rückführfeld (14) als etwa einen 4 Grad Temperaturanstieg übersteigend prognostiziert wird.

4. System nach Anspruch 1, wobei das System angepasst ist, um an ein bestehendes monopolares elektrochirurgisches System wirkgekoppelt zu werden.

5. System nach Anspruch 1, wobei die Vorrichtung zwischen der Rückführelektrode (14) und dem Generator (10) wirkgekoppelt ist.

6. System nach Anspruch 1, wobei das Warnsignal vor Zuständen erzeugt wird, die eine Verbrennung beim Patienten hervorrufen könnten.

## Revendications

1. Système pour prédire la température sous une électrode de retour (14) dans un système électrochirurgical monopolaire, comprenant:
un appareil de détection (40) apte à être connecté à une source de puissance (48) comportant:
un circuit analogique (50a) comportant:
une résistance de génération de chaleur (44a, 44b) montée en série entre un générateur (10) et ladite électrode de retour (14) et
un capteur de température (46) configuré pour détecter un changement de température de ladite résistance (44a, 44b);
où ladite résistance de génération de chaleur (44a, 44b) et ledit capteur de température (46) sont en communication thermique;
un comparateur (50b) configuré pour comparer ledit changement de température avec une valeur de seuil; et
un circuit d'alarme (50) configuré pour produire un signal d'avertissement;
où les propriétés de chauffage et de refroidissement dudit circuit analogique (50a) sont proportionnelles aux propriétés de chauffage et de refroidissement de la peau à l'électrode de retour (14), où ledit appareil de détection (40) est configuré pour prédire la température de la peau du patient à ladite électrode de retour (14) dudit système électrochirurgical monopolaire sans venir en contact avec le patient, et où ledit circuit d'alarme (50) est configuré pour alerter l'utilisateur lorsque la température prédite de la peau dépasse la valeur de seuil.

2. Appareil selon la revendication 1, dans lequel ladite source de puissance (48) comprend une alimentation portable.

3. Système selon la revendication 1, dans lequel ledit signal d'avertissement est produit lorsqu'il est prédit que la température sous le coussinet de retour (14) dépasse une augmentation de température d'environ 4 degrés.

4. Système selon la revendication 1, dans lequel le système est conçu pour être couplé fonctionnellement à un système électrochirurgical monopolaire existant.

5. Système selon la revendication 1, dans lequel l'appareil est fonctionnellement couplé entre l'électrode de retour (14) et le générateur (10).

6. Système selon la revendication 1, dans lequel ledit signal d'avertissement est produit avant des conditions qui peuvent entraîner une brûlure du patient.
